# EUROPEAN PATENT APPLICATION

(11) **EP 3 330 297 A1**
(43) Date of publication of application: **06.06.2018**
(21) Application number: 16832738.5
(22) Date of filing: 15.07.2016
(51) Int. Cl.: C08F 8/12

(54) **METHOD OF USING AQUEOUS SOLUTION OF POLYMER COMPRISING VINYLAMINE UNITS**

(30) Priority: 31.07.2015 JP 2015152725
(71) Applicant: Mitsubishi Chemical Corporation, Tokyo 100-8251 (JP)
(72) Inventor: ISHII Akihiro, Tokyo 100-8251 (JP); MORI Yasuharu, Tokyo 100-8251 (JP)
(74) Representative: Ter Meer Steinmeister & Partner
(86) International application number: PCT/JP2016/071026
(87) International publication number: WO 2017/022459

(57) **Abstract**

A method comprising producing N-vinylformamide, polymerizing a monomer ingredient comprising the N-vinylformamide to produce a polymer comprising N-vinylformamide units, hydrolyzing the polymer using an acid or a base to produce an aqueous solution of a polymer comprising vinylamine units, and using the aqueous solution, wherein the storage period (2) from immediately after the production of the polymer to just before initiation of the production of the aqueous solution of a polymer comprising vinylamine units is made longer than the storage period (3) from immediately after the production of the aqueous solution to just before the use.

## Description

### TECHNICAL FIELD

The present invention relates to a method of using an aqueous solution of a vinylamine unit-containing polymer.

This application is based upon and claims the benefit of priority of the prior Japanese Patent Application No. 2015-152725 filed in Japan on July 31, 2015, the entire contents of which arc incorporated herein by reference.

### BACKGROUND ART

Vinylamine unit-containing polymers arc widely utilized as aggregating agents, papermaking chemicals, fiber treatment agents, paint additives, and the like, and polymers having various molecular weights over a wide range from high molecular weight polymers having a molecular weight of 4,000,000 or more to low molecular weight polymers having a molecular weight of 100,000 or less arc utilized depending on the application.

As methods of manufacturing vinylamine unit-containing polymers, various manufacture methods are known and a method in which an N-vinylformamide unit-containing polymer is (partially) hydrolyzed to convert the formamide group of the N-vinylformamide unit to an amino group is useful from the industrial viewpoint

As a method of manufacturing N-vinylformamide which is a starting material of an N-vinylformamide unit-containing polymer, the following methods arc known.
(1) A method in which N-methoxyethylformamide is thermally decomposed to obtain N-vinylformamide (Patent Literature 1).
(2) A method in which N-cyanoethylformamide is thermally decomposed to obtain N-vinylformamide (Patent Literature 2).
   N-vinylformamide exhibits low stability as loss due to a decomposition or polymerization reaction in any of acids, alkalis, heat, radicals, or the like occurs, and as a result, the purity of N-vinylformamide decreases. N-vinylformamide with a decreased purity causes a decrease in the polymerization rate and a decrease in the molecular weight of the polymer.
   As a method of enhancing the stability of N-vinylformamide, the following methods are known.
(3) A method in which a thiourea is added to N-vinylformamide (Patent Literature 3).
(4) A method in which N-vinylformamide is preserved in an inert gas atmosphere and the pH is adjusted (Patent Literature 4).
(5) A method in which a specific alcohol is added to N-vinylformamide (Patent Literature 5).
(6) A method in which a specific monomer is added to N-vinylformamide as a stabilizer (Patent Literature 6).
(7) A method in which formic acid is added to N-vinylformamide (Patent Literature 7).
   With regard to a method of manufacturing a vinylamine unit-containing polymer to be a final product, the following methods are known.
(8) A method in which a powdery N-vinylformamide unit-containing polymer is obtained, then prepared into an aqueous solution, and hydrolyzed in the presence of an acid or a base, thereby obtaining an aqueous solution of a vinylamine unit-containing polymer (Patent Literature 8).
(9) A method in which a gel-like N-vinylformamide unit-containing polymer is obtained and hydrolyzed by being directly mixed with a basic aqueous solution, thereby obtaining a vinylamine unit-containing polymer (Patent Literature 9).
(10) A method in which a powdery N-vinylformamide unit-containing polymer is obtained and then hydrolyzed by being directly mixed with an acidic or basic aqueous solution, thereby obtaining a powdery vinylamine unit-containing polymer (Patent Literature 10).
   Vinylamine unit-containing polymers arc finally used in the form of an aqueous solution in number of cases. However, an aqueous solution of a vinylamine unit-containing polymer docs not exhibit high stability and a phenomenon is observed that the viscosity of the aqueous solution decreases as the molecular chain is cleaved. In the case of using a vinylamine unit-containing polymer in a papermaking additive, an aggregating agent, and the like to utilize the crosslinking, adsorbing, and aggregating effects thereof, it is more advantageous as the vinylamine unit-containing polymer has a higher molecular weight and a decrease in the molecular weight leads to a decrease in the performance. The above phenomenon is more remarkable as the molecular weight of the vinylamine unit-containing polymer is higher and the appearance viscosity of the aqueous solution is lower, that is, the aqueous solution is a more dilute solution.
   As a method of enhancing the stability of an aqueous solution of a vinylamine unit-containing polymer, the following method is known.
(11) A method in which an N-vinylformamide unit-containing polymer is hydrolyzed with an acid, a C₁-C₆ alcohol is added to the aqueous solution of a vinylamine unit-containing polymer thus obtained, formic acid produced by hydrolysis is esterified, and this ester is distilled off (Patent Literature 11).

Meanwhile, long-tenn preservation (storage, transportation, and the like) is required in number of cases when vinylamine unit-containing polymers are actually manufactured and put to practical use. It is common practice, for example, to stockpile the vinylamine unit-containing polymer product for shipping and ordering to remote areas and to transport N-vinylformamide to be a starting material and to collectively manufacture the polymers by using this in order to improve the manufacture efficiency.

Such long-term preservation is accompanied by a problem of deterioration such as a decrease in the viscosity of the aqueous solution.

It is difficult to prevent all side reactions even if a stabilizer is added as described in Patent Literature 3, Patent Literature 5, Patent Literature 6, and Patent Literature 7 as a countermeasure for deterioration of N-vinylformamide. In addition, the product cost increase as a stabilizer is added. The method described in Patent Literature 4 also has insufficient effects, is restrictive in terms of industrial handling, and is not economical.

Meanwhile, as described in Patent Literature 10, the problem of deterioration is suppressed when a vinylamine unit-containing polymer is manufactured in the form of a powder but not an aqueous solution and dissolved immediately before use. However, it cannot be said that the effect is sufficient.

In Patent Literature 8 and Patent Literature 9. the stability of the polymer is not described.

The method described in Patent Literature 11 can enhance the stability of the aqueous solution, but the manufacturing operation is complicated and an increase in the product cost accompanying this is a problem.

### CITATION LIST

### PATENT LITERATURE

Patent Literature 1: JP 61-97309 A
Patent Literature 2: JP 61-134359 A
Patent Literature 3: JP 61-289068 A
Patent Literature 4: JP 63-264559 A
Patent Literature 5: JP 10-1462 A
Patent Literature 6: JP 6-41034 A
Patent Literature 7: JP 6-122660 A
Patent Literature 8: JP 2004-75814 A
Patent Literature 9: JP 2006-257287 A
Patent Literature 10: JP 2011-162671 A
Patent Literature 11: JP 10-501561 W

### SUMMARY OF THE INVENTION

### PROBLEM TO BE SOLVED BY THE INVENTION

The invention provides a method capable of suppressing the influence of preservation conducted during the period from the manufacture of N-vinylformamide to the manufacture of an aqueous solution of a vinylamine unit-containing polymer via an N-vinylformamide unit-containing polymer and the use thereof on the quality of the aqueous solution of a vinylamine unit-containing polymer and stably using the aqueous solution of a vinylamine unit-containing polymer.

### MEANS FOR SOLVING PROBLEM

As a result of intensive investigations in view of the above problems, the inventors of the invention have found out that an intermediate N-vinylfoimamide unit-containing polymer to be produced when manufacturing a vinylamine unit-containing polymer exhibits stability overwhelmingly higher than that of N-vinylformamide and the vinylamine unit-containing polymer and found out the most reasonable method of using an aqueous solution of a vinylamine unit-containing polymer in consideration of this difference in stability.

The invention has the following aspects.
<1> A method of using an aqueous solution of a viny lamine unit-containing polymer, the method including:
   manufacturing N-vinylformamide;
   manufacturing an N-vinylformamide unit-containing polymer by polymerizing a monomer component containing the N-vinylformamide:
      manufacturing an aqueous solution of a vinylamine unit-containing polymer by hydrolyzing the N-vinylformamide unit-containing polymer by using an acid or a base and water; and
      setting a preservation period (2) of the N-vinylformamide unit-containing polymer from immediately after manufacture of the N-vinylformamide unit-containing polymer to immediately before start of manufacture of the aqueous solution of a vinylamine unit-containing polymer to be longer than a preservation period (3) of the aqueous solution of a vinylamine unit-containing polymer from immediately after manufacture of the aqueous solution of a vinylamine unit-containing polymer to immediately before use of the aqueous solution of a vinylamine unit-containing polymer when using the aqueous solution of a vinylamine unit-containing polymer.
<2> A method of using an aqueous solution of a vinylamine unit-containing polymer, the method including:
   manufacturing N-vinylformamide;
   manufacturing an N-vinylformamide unit-containing polymer by polymerizing a monomer component containing the N-vinylformamide;
   manufacturing an aqueous solution of a vinylamine unit-containing polymer by hydrolyzing the N-vinylformamide unit-containing polymer by using an acid or a base and water; and
   setting a preservation period (2) of the N-vinylformamide unit-containing polymer from immediately after manufacture of the N-vinylformamide unit-containing polymer to immediately before start of manufacture of the aqueous solution of a vinylamine unit-containing polymer to be longer than a preservation period (1) of the N-vinylformamide from immediately after manufacture of the N-vinylformamide to immediately before start of manufacture of the N-vinylformamide unit-containing polymer and a preservation period (3) of the aqueous solution of a vinylamine unit-containing polymer from immediately after manufacture of the aqueous solution of a vinylamine unit-containing polymer to immediately before use of the aqueous solution of a vinylamine unit-containing polymer when using the aqueous solution of a vinylamine unit-containing polymer.
<3> The use method according to <1>, in which a sum of the preservation periods (2) and (3) is 10 days or longer.
<4> The use method according to <2>, in which a sum of the preservation periods (1), (2), and (3) is 10 days or longer.
<5> The use method according to any one of <1> to <4>, in which a powdery substance is manufactured as the N-vinylformamide unit-containing polymer by polymerizing the monomer component and then drying a resultant substance and preserved during the preservation period (2).
<6> The use method according to any one of <1 > to <5>, in which a preservation atmosphere of the N-vinylformainide unit-containing polymer is a temperature of 25°C or higher in at least a part of the preservation period (2).
<7> The use method according to any one of <1> to <6>, in which a weight average molecular weight of the N-vinylformamide unit-containing polymer is 100,000 or more.
<8> The use method according to any one of <1> to <7>, in which the hydrolysis is conducted in an aqueous solution of the N-vinylformamide unit-containing polymer in the presence of a strong base.
<9> The use method according to any one of <1> to <8>, in which a powdery substance is manufactured as the N-vinylformamide unit-containing polymer and transported to another place while applying a vibration of from 0.1 to 400 Hz to the substance during the preservation period (2), the N-vinylformamide unit-containing polymer is prepared into an aqueous solution, the hydrolysis is conducted in the aqueous solution, and a sum of the preservation periods (1). (2), and (3) is 10 days or longer.

### EFFECT OF THE INVENTION

According to the use method of the invention, it is possible to suppress the influence of preservation conducted during the period from the manufacture of N-vinylfonnamide to the manufacture of an aqueous solution of a vinylamine unit-containing polymer via an N-vinylformamide unit-containing polymer and the use thereof on the quality of the aqueous solution of a vinylamine unit-containing polymer and to stably use the aqueous solution of a vinylamine unit-containing polymer.

### MODE(S) FOR CARRYING OUT THE INVENTION

The following term definitions apply throughout this specification and the claims.

The term "monomer" means a compound having an ethylenically unsaturated bond.

The term "unit" in a polymer means a constitutional unit derived from a monomer formed by polymerization of a monomer or a constitutional unit in which a part of the constitutional unit is converted to another structure by treating a polymer.

The term "crude N-vinylformamide" means a mixture of N-vinylformamide and impurities derived from starting materials.

The term "N-vinylformamide unit-containing polymer" means a homopolymer composed of an N-vinylformamide unit or a copolymer having an N-vinylformamide unit and constitutional units other than this (provided that a copolymer having an N-vinylformamide unit and a vinylamine unit is excluded).

The term "vinylamine unit-containing polymer" means a homopolymer composed of a vinylamine unit or a copolymer having a vinylamine unit and constitutional units other than this. The vinylamine unit may be in the form of a salt.

The term "strong acid" means a compound having an acid dissociation constant pKa of 0 or lower in an aqueous solution at 25°C.

The term "strong base" means a compound having a base dissociation constant pKb of 0 or lower in an aqueous solution at 25°C.

The term "(meth)acrylic acid" is a generic term for acrylic acid and methacrylic acid.

The term "(meth)acrylic acid ester" is a generic term for an acrylic acid ester and a meth acrylic acid ester.

The term "(meth)acrylonitrile" is a generic term for acrylonitrile and methacrylonitrile.

The term "(meth)acrylamide" is a generic tenn for acrylamide and methacrylamide.

The term "manufacture" means to synthesize an intended substance by a reaction (thermal decomposition, polymerization, hydrolysis, and the like) and to subject the crude product thus produced to an additional treatment (purification, drying (powdering), and the like) in some cases to form the crude product into a form which can be used as a finished product.

The term "from immediately after the manufacture of N-vinylformamide to immediately before the start of manufacture of an N-vinylformamide unit-containing polymer" means the period from the time point at which N-vinylformamide is manufactured (the time point at which N-vinylformamide is formed into a form to be used in the manufacture of an N-vinylformamide unit-containing polymer) to the time point at which the manufacture of an N-vinylformamide unit-containing polymer is starred.

The term "from immediately after the manufacture of an N-vinylformamide unit-containing polymer to immediately before the start of manufacture of an aqueous solution of a vinylamine unit-containing polymer" means the period from the time point at which an N-vinylformamide unit-containing polymer is manufactured (the time point at which the N-vinylformamide unit-containing polymer is prepared into a form to be used in the manufacture of an aqueous solution of a vinylamine unit-containing polymer) to the time point at which the manufacture of an aqueous solution of a vinylamine unit-containing polymer is started.

The term "from immediately after the manufacture of an aqueous solution of a vinylamine unit-containing polymer to immediately before the use of the aqueous solution of a vinylamine unit-containing polymer" means the period from the time point at which an aqueous solution of an N-vinylformamide unit-containing polymer is manufactured (the time point at which an aqueous solution of a vinylamine unit-containing polymer is prepared into a form to be used) to the time point at which the use of an aqueous solution of a vinylamine unit-containing polymer is started.

Examples of the use method of the invention may include a method having the following steps (I) to (VIII).
(I) A step of obtaining N-vinylformamide.
(II) A step of preserving the N-vinylformamidc between the step (I) and the step (III) if necessary.
(III) A step of polymerizing a monomer component containing the N-vinylformamide to obtain an N-vinylformamide unit-containing polymer.
(VI) A step of drying the N-vinylformamide unit-containing polymer to form it into a powdery shape between the step (III) and the step (V) if necessary.
(V) A step of preserving the N-vinylformamide unit-containing polymer.
(VI) A step of hydrolyzing the N-vinylformamide unit-containing polymer by using an acid or a base and water to obtain an aqueous solution of a vinylamine unit-containing polymer.
(VII) A step of preserving the aqueous solution of a vinylamine unit-containing polymer between the step (VI) and the step (VIII) if necessary.
(VIII) A step of using the aqueous solution of a vinylamine unit-containing polymer.

### (Step (I))

Examples of a method of manufacturing an N-vinylformamide monomer may include the following methods.
(1) A method in which N-methoxyethylformamide is thermally decomposed to obtain N-vinylformamide (Patent Literature 1).
(2)A method in which N-cyanoethylformamide is thermally decomposed to obtain N-vinylformamide (Patent Literature 2).
(3) A method in which N-vinylformamide is obtained from ethylenebisformamide.

### (Step (II))

As deseribed above, N-vinylformamide exhibits low stability as loss duc to a decomposition or polymerization reaction in any of acids, alkalis, heat, or radicals, occurs. In addition, N-vinylformamide has problems such as undesirable polymerization in addition to loss due to decomposition at a high temperature. Hence, it is preferable that the N-vinylformamide obtained in the step (I) is polymerized as soon as possible mto an N-vinylformamide unit-containing polymer.

The preservation period of N-vinylformamide, namely, the preservation period (1) of N-vinylformamide from immediately after the manufacture of N-vinylformamide in the step (I) to immediately before the start of manufacture of the N-vinylformamide unit-containing polymer in the step (III) is set to be shorter than the preservation period (2) to be described later, and it is more preferable as the preservation period (1) is shorter. The preservation period (I) is preferably from 0 to 10 days, more preferably 0 to 5 days, and may be 0 day.

In the case of preserving N-vinylformamide, it is preferable to preserve N-vinylformamide by being kept cool even for a short period of time. The temperature to keep N-vinylformamide cool is preferably 10°C or lower and more preferably from 5°C to 10°C. However, to keep N-vinylformamide cool requires a special facility, and a great increase in the cost is caused in the case of conducting long-distance transportation or long-term preservation. Hence, it is preferable to polymerize N-vinylformamide into an N-vinylformamide unit-containing polymer exhibiting higher stability as soon as possible.

### (Step (III))

The monomer component contains at least N-vinylformamidc.

The monomer component may contain monomers other than N-vinylformamide. Examples of other monomers may include (meth)acrylic acid, satls of (meth)acrylic acid, (meth)acrylic acid esters, (meth)acrylonitrile, (meth)acrylamide, N-alkyl(meth)acrylamides, N,N-dialkyl(meth)acrylamides, dialkylaminoethyl(meth)acrylamides, salts or quaternized compounds of dialkylaminoethyl(meth)acrylamides, dialkylaminopropyl(meth)acrylamides, salts or quaternized compounds of dialkylaminopropyl(meth)acrylamides, diacetone acrylamide, N-vinylpyrrolidone. N-vinylcaprolactam, and vinyl acctatc.

The proportion of N-vinylformamide is usually 5 mol% or more, preferably 10 mol% or more, more preferably 50 mol% or more, and still more preferably from 70 to 100 mol% with respect to the sum (100 mol%) of all the monomer components. The characteristics of N-vinylformamide arc further exerted as the proportion thereof is higher. The proportion of each monomer is reflected as the proportion of each constitutional unit in the N-vinylformamide unit-containing polymer or the vinylamine unit-containing polymer.

Examples of a method of polymerizing the monomer component may include an aqueous solution polymerization method, an aqueous solution adiabatic polymerization method, a reversed phase suspension polymerization method, an emulsion polymerization method, and a sheet-like photopolymerization method.

Polymerization of the monomer component is usually conducted at a pH of from 5 to 9. The hydrolysis of N-vinylformamide is suppressed when the pH is in a range of from 5 to 9.

The polymerization temperature varies depending on the polymerization method, but it is usually from 0°C to 110°C and preferably from 0°C to 100°C.

When polymerizing the monomer component, an initiator, a solvent, other known additives, and the like may be used if necessary.

The initiator may be appropriately selected from known initiators depending on the polymerization method to be employed. Examples of the initiator may include an azo-bascd initiator, a redox-based initiator, a peroxide-based initiator, and a photoinitiator.

The solvent may be appropriately selected from known solvents depending on the polymerization method to be employed. Examples of the solvent may include water and a hydrocarbon-based solvent.

Examples of other additives may include a gel quality improver (polyalkylene glycols or the like), a pH control chemical (phosphoric acid or the like), an inorganic salt, a chain transfer agent, an emulsifier (dispersion stabilizer), and a sensitizer.

It is more preferable as the molecular weight of the N-vinylformamide unit-containing polymer is higher since it is more advantageous as the molecular weight of the vinylamine unit-containing polymer to be contained in the aqueous solution of a vinylamine unit-containing polymer to be obtained in the subsequent step is higher in terms of performance.

The molecular weight of the N-vinylformamide unit-containing polymer is preferably 100,000 or more and more preferably 200,000 or more as a weight average molecular weight. The upper limit of the weight average molecular weight is not particularly limited, but it is preferably 3,000,000 or less since the viscosity when being prepared into an aqueous solution increases as the molecular weight increases and the handling property becomes poor. The molecular weight of the N-vinylformamide unit-containing polymer is preferably from 100,000 to 3,000,000 and more preferably from 200,000 to 3,000,000.

The weight average molecular weight of the N-vinylformamide unit-containing polymer is measured according to the (measurement of molecular weight of N-vinylformamide unit-containing polymer and vinylamine unit-containing polymer) to be described in Examples later.

### (Step (IV))

The state of the N-vinylformamide unit-containing polymer to be obtained in the step (III) is a massive aqueous gel in the case of aqueous solution adiabatic polymerization and sheet-like photopolymerization, it is a particulate gel dispersed in a nonaqueous solvent in the case of reversed phase suspension polymerization, it is an aqueous solution in the case of an aqueous solution polymerization method, and it is an emulsion in the case of an emulsion polymerization method.

The N-vinylformamidc unit-containing polymer may be subjected to the step (V) as it is, but the N-vinylformamide unit-containing polymer may be subjected to the step (V) after being formed into a powdery shape through drying. By being formed into a powdery shape through drying, the stability of the N-vinylformamide unit-containing polymer increases. In addition, the N-vinylformamide unit-containing polymer has a larger specific surface area when being formed into a powdery shape, and it can be thus uniformly dissolved or dispersed in a shorter time when being dissolved or dispersed in water again for the hydrolysis treatment in the step (VI). There is also an advantage that the transportation is further facilitated.

Drying is usually conducted at from 50°C to 140°C. It is preferable that the volatile component in the powdery substance after drying is from 0.1 to 12 mass% of the total mass from the viewpoint of ease of handling.

The N-vinylformamidc unit-containing polymer may be subjected to a pulverization treatment in either or both of before and after drying.

An auxiliary may be used when forming the polymer into a powder (when conducting drying or pulverization treatment) in order to suppress adhesion of the particles to each other. Examples of the auxiliary may include various kinds of oils such as polyalkylene glycols and silicone oil and a surfactant.

It is preferable that the particle diameter of the powdery N-vinylformamide unit-containing polymer obtained in the step (IV) is usually in a range of from 4 mesh-pass to 500 mesh-on.

### (Step (V))

The preservation of the N-vinylformamide unit-containing polymer is conducted in a state of a massive aqueous gel obtained by aqueous solution adiabatic polymerization or sheet-like photopolymerization in the step (III); a state of a particulate gel dispersed in a nonaqueous solvent obtained by reversed phase suspension polymerization in the step (III); and a state of a powder obtained in the step (IV).

The preservation of the N-vinylformamide unit-containing polymer includes storage and transportation. In other words, only storage of the N-vinylformamide unit-containing polymer may be conducted, only transportation thereof may he conducted, or both storage and transportation thereof may be conducted in the step (V).

The preservation period of the N-vinylformamide unit-containing polymer, namely, the preservation period (2) of the N-vinylformamide unit-containing polymer from immediately after the manufacture of the N-vinylformamide unit-containing polymer to immediately before the start of manufacture of the aqueous solution of a vinylamine unit-containing polymer is the longest among the preservation period (1) described above, the preservation period (2), and the preservation period (3) to be described later. In other words, the N-vinylformamide unit-containing polymer is preserved for the longest period of time among N-vinylformamide, the N-vinylformamide unit-containing polymer, and the aqueous solution of a vinylamine unit-containing polymer. The preservation period (3) is preferably from 0 to 10 days, more preferably 0 to 5 days, and may be 0 day.

The stability of the N-vinylformamide unit-containing polymer is the highest among N-vinylformamide, the N-vinylformamidc unit-containing polymer, and the aqueous solution of a vinylamine unit-containing polymer. Hence, the aqueous solution of a vinylamine unit-containing polymer to be used in the step (VIII) is less deteriorated from the intended quality (viscosity of aqueous solution, molecular weight of vinylamine unit-containing polymer, and the like) by maximizing the proportion of the preservation period (2) in the sum of the preservation periods (1), (2), and (3) (hereinafter also referred to as the "total preservation period").

In a case in which the step (IV) is not conducted but the N-vinylformamide unit-containing polymer obtained in the step (III) (N-vinylformamide unit-containing polymer not powdered) is subjected to the step (V) as it is, the preservation period (2) is a period from the time point at which the N-vinylformamide unit-containing polymer is obtained in the step (III) to the time point at which the hydrolysis treatment is started in the step (VI).

In a case in which the step (IV) is conducted and the powdery N-vinylformamide unit-containing polymer obtained in the step (IV) is subjected to the step (V), the preservation period (2) is a period from the time point at which the powdery N-vinylformamide unit-containing polymer is obtained in the step (IV) to the time point at which the hydrolysis treatment is started in the step (VI).

The proportion of the preservation period (2) to the total preservation period is preferably from 60% to 100% and more preferably from 80% to 100%.

The total preservation period is preferably 10 days or longer and more preferably 15 days or longer. The effect of the invention is more remarkable as the total preservation period is longer. In other words, the deterioration progress of N-vinylformamide or the aqueous solution of a vinylamine unit-containing polymer is promoted more than that of the N-vinylformamide unit-containing polymer and the difference in stability among these is more remarkable as the total preservation period is longer.

The total preservation period is preferably 180 days or shorter from the viewpoint of suppressing the deterioration.

In at least a part of the preservation period (2), the preservation atmosphere of the N-vinylformamide unit-containing polymer is a temperature of preferably 25°C or higher and more preferably from 25°C to 40°C. The effect is more remarkable as the temperature of preservation atmosphere is 25°C or higher. In other words, the deterioration progress of N-vinylformamide or the aqueous solution of a vinylamine unit-containing polymer is promoted more than that of the N-vinylformamide unit-containing polymer and the difference in stability among these is more remarkable as the temperature at the time of preservation is higher. At least a part of the preservation period (2) is preferably from 10 to 140 days and more preferably from 15 to 110 days.

### (Step (VI))

The hydrolysis of the N-vinylformamide unit-containing polymer is conducted in the presence of water. Specifically, the hydrolysis is conducted in a state of a massive aqueous gel obtained by aqueous solution adiabatic polymerization or sheet-like photopolymerization in the step (III); a state of a particulate gel dispersed in a nonaqueous solvent obtained by reversed phase suspension polymerization in the step (III): and a state in which the powdery N-vinylformamide unit-containing polymer obtained in the step (IV) is dissolved or dispersed in water. A mixed solvent containing an organic solvent other than water may be used. A salt may be contained in water.

By hydrolyzing the N-vinylformamide unit-containing polymer, a part or all of the formamide groups (-NHC(=O)H) in the N-vinylformamide unit-containing polymer is converted to an amino group to form a vinylamine unit-containing polymer, and an aqueous solution of a vinylamine unit-containing polymer is thus obtained.

The hydrolysis of the N-vinylformamide unit-containing polymer is conducted by using an acid or a base. It is preferable to conduct the hydrolysis in an aqueous solution of the N-vinylformamide unit-containing polymer in the presence of a strong acid or a strong base. The strong acid is preferably a monovalent mineral acid such as hydrochloric acid or nitric acid. The strong base is preferably lithium hydroxide, sodium hydroxide, potassium hydroxide, and the like. It is more preferable to use a strong base from the viewpoint of suppressing corrosion of the apparatus.

The rate of hydrolysis of the formamide groups in the N-vinylformamide unit-containing polymer is preferably 10 mol% or more of all the formamide groups before hydrolysis. It is concerned that it is difficult to accurately control the rate of hydrolysis in a case in which the target rate of hydrolysis is too low. The upper limit of the rate of hydrolysis is preferably 80 mol% or less of all the formamide groups before hydrolysis. An excessive amount of a strong acid or a strong base is required in the case of hydrolyzing more than 80 mol% of the formamide groups in the N-vinylformamide unit-containing polymer. The rate of hydrolysis of the formamide groups in the N-vinylformamide unit-containing polymer is preferably from 10 to 80 mol%.

The temperature at which hydrolysis of the N-vinylformamide unit-containing polymer is conducted is preferably from 50°C to 100°C. The hydrolysis reaction is promoted and a desired rate of hydrolysis is obtained in a relatively short time when the temperature at which hydrolysis of the N-vinylformamide unit-containing polymer is conducted is 50°C or higher. A decrease in the molecular weight and insolubilization by heat are not caused and a high quality vinylamine unit-containing polymer is obtained when the temperature at which hydrolysis of the N-vinylformamide unit-containing polymer is conducted is 100°C or lower.

The molecular weight of the vinylamine unit-containing polymer contained in the aqueous solution of a vinylamine unit-containing polymer is preferably 100,000 or more and more preferably 200,000 or more as a weight average molecular weight. The upper limit of the weight average molecular weight is not particularly limited, but it is preferably 3,000,000 or less since the viscosity when being prepared into an aqueous solution increases as the molecular weight increases and the handling property becomes poor. The molecular weight of the vinylamine unit-containing polymer is preferably from 100,000 to 3.000.000 and more preferably from 200,000 to 3.000.000.

The aqueous solution of a vinylamine unit-containing polymer usually contains formic acid produced by hydrolysis of the formamide group in the N-vinylformamide unit-containing polymer and ammonia, formic acid, and acetaldehyde produced by hydrolysis of N-methoxyethylformamide.

It is preferable to remove acetaldehyde by a known method since it causes insolubilization of the vinylamine unit-containing polymer by crosslinking at the time of the hydrolysis treatment. Specific examples thereof may include a method in which acetaldehyde is reduced with a reducing agent and a method in which acetaldehyde is convened into an oxime through the reaction with hydroxylamine.

### (Step (VII))

As described above, the aqueous solution of a vinylamine unit-containing polymer exhibits lower stability than the N-vinylformamidc unit-containing polymer and a decrease in the molecular weight of the vinylamine unit-containing polymer and a decrease in the viscosity of the aqueous solution are likely to occur at the time of preservation of the aqueous solution. Hence, it is preferable to use the aqueous solution of a vinylamine unit-containing polymer obtained in the step (VI) as soon as possible.

The preservation period of the aqueous solution of a vinylamine unit-containing polymer, namely, the preservation period (3) of the aqueous solution of a vinylamine unit-containing polymer from immediately after the manufacture of the aqueous solution of a vinylamine unit-containing polymer in the step (VI) to immediately before the use of the aqueous solution of a vinylamine unit-containing polymer in the step (VIII) is set to be shorter than the preservation period (2), and it is more preferable as the preservation period (3) is shorter. The preservation period (3) is preferably 10 days or shorter, more preferably 5 days or shorter, and may be 0 day.

In the case of preserving the aqueous solution of a vinylamine unit-containing polymer, the preservation temperature is preferably 40°C or lower and more preferably 30°C or lower. The lower limit value is preferably 0°C or higher.

### (Step (VIII))

The method of using the aqueous solution of a vinylamine unit-containing polymer is not particularly limited, and a known method can be employed depending on the application. Examples of the application of the aqueous solution of a vinylamine unit-containing polymer may include an aggregating agent, a papermaking chemical, a fiber treatment agent, a paint additive, and a petroleum recovery chemical.

In the use method of the invention, preservation includes storage and transportation. Particularly the transportation includes transportation forms such as ships, railroads, and airplanes, but a certain vibration is applied to the product in any form. It has been revealed mass transfer in the product actively occurs by the vibration and the rate of deterioration is accelerated in this case. Meanwhile, it has been revealed that the powdery N-vinylformamide unit-containing polymer exhibits significantly high stability even during the transportation under vibrations. The difference in stability is more remarkable than that during usual storage when the stability of the powdery N-vinylformamide unit-containing polymer is compared to the stability of N-vinylformamide and the aqueous solutions of a vinylamine unit-containing polymer, and it is thus significantly advantageous to maximize the preservation period of the powdery N-vinylformamide unit-containing polymer in a case in which transportation is essential. The frequency of vibration to be applied during transportation is preferably from 0.1 to 400 Hz and more preferably from 1 to 200 Hz. The vibration is not almost generated when the frequency of vibration is 0.1 Hz or less, and a frequency of vibration of 400 Hz or more is not realistic since it is great as a general frequency of vibration during transportation.

In the use method of the invention described above, the N-vinylformamide unit-containing polymer is preserved over the longest period of time among three forms of N-vinylformamide, an N-vinylformamide unit-containing polymer, and an aqueous solution of a vinylamine unit-containing polymer in the period from the manufacture of N-vinylformamide to the manufacture and use of the aqueous solution of a vinylamine unit-containing polymer via the N-vinylformamide unit-containing polymer.

N-vinylformamide is induced into a vinylamine unit-containing polymer through polymerization and hydrolysis. However, in the case of preserving the vinylamine unit-containing polymer having a high molecular weight in an aqueous solution state, a phenomenon that the viscosity of the aqueous solution decreases is often observed and the average molecular weight of the vinylamine unit-containing polymer also decreases. Meanwhile, the N-vinylformamide unit-containing polymer which is an intermediate before obtaining the vinylamine unit-containing polymer is nonionic and an aqueous solution thereof is also neutral, and thus the N-vinylformamide unit-containing polymer is more stable than the vinylamine unit-containing polymer. In addition, the stability of the N-vinylformamide unit-containing polymer is outstandingly higher than N-vinylformamide which is easily decomposed by heat or a radical.

Hence, by maximizing the proportion of the preservation period (2) in the state of the N-vinylformamide unit-containing polymer in the total preservation period, the proportion of the preservation periods (1) and (3) is relatively lowered and it is possible to efficiently suppress the deterioration (decrease in purity) of N-vinylformamide and a decrease in polymerization rate accompanying this, a decrease in the molecular weight of the polymer, and the cleavage of the molecular chain of the vinylamine unit-containing polymer in the aqueous solution of a vinylamine unit-containing polymer (decrease in molecular weight of polymer and decrease in viscosity of aqueous solution). Hence, it is possible to use the aqueous solution of a vinylamine unit-containing polymer having small loss due to deterioration and to sufficiently exert the performance of the vinylamine unit-containing polymer without conducting complicated manufacturing operations.

In particular, the effect of decreasing the loss due to deterioration is more remarkable and the advantage of the invention is greater as the molecular weight of the vinylamine unit-containing polymer is higher, the total preservation period is longer, the temperature at the preservation place is higher, or the rate of hydrolysis of the vinylamine unit-containing polymer is lower.

In the use method of the invention, it is preferable that a powdery substance is manufactured as the N-vinylformamide unit-containing polymer and transported to another place (for example, as close as possible to the place at which the aqueous solution of a vinylamine unit-containing polymer is used) during the preservation period (2), the N-vinylformamide unit-containing polymer is prepared into an aqueous solution, the hydrolysis is conducted in the aqueous solution, and the sum of the preservation periods (1), (2), and (3) (total preservation period) is 10 days or longer. This makes the most stably use of an aqueous solution of a vinylamine unit-containing polymer to be obtained from N-vinylformamide including transportation and preservation possible. It is also possible to cut down the transportation cost by obtaining an aqueous solution of a vinylamine unit-containing polymer in the vicinity of a consumer and subjecting it to the use by the consumer.

Another aspect of the invention includes a method of manufacturing an aqueous solution of a vinylamine unit-containing polymer. Examples of the method of manufacturing an aqueous solution of a vinylamine unit-containing polymer may include a method having the following steps (I') to (VII').
(I') A step of obtaining N-vinylformamide.
(II') A step of preserving the N-vinylformamide between the step (I') and the step (III') if necessary.
(III') A step of polymerizing a monomer component containing the N-vinylformamide to obtain an N-vinylformamide unit-containing polymer.
(VI') A step of drying the N-vinylformamide unit-containing polymer to form it into a powdery shape between the step (III') and the step (V') if necessary.
(V') A step of preserving the N-vinylformamide unit-containing polymer.
(VI') A step of hydrolyzing the N-vinylformamide unit-containing polymer by using an acid or a base and water to obtain an aqueous solution of a vinylamine unit-containing polymer.
(VII') A step of preserving the aqueous solution of a vinylamine unit-containing polymer between the step (VI') and the step (VIII') if necessary. The steps (I') to (VII') are the same as the steps (I) to (VII).

### EXAMPLES

Next, the invention will be described in more detail with reference to Examples, but the invention is not limited to the following Examples unless it goes beyond its gist.

Incidentally, the physical properties of the polymers were measured by the following methods in Examples and Comparative Examples.

### (Measurement of Purity of N-vinylformamide in Crude N-vinylformamide)

A predetermined amount of crude N-vinylformamide was taken in a 25 mL volumetric flask and diluted with a 0.01 mol/L aqueous solution of disodium hydrogenphosphate so as to have a concentration of 0.01 mass%. The diluted solution was subjected to analysis by liquid chromatography (LC) using the following apparatus under the following conditions to measure the content of N-vinylformamide in crude N-vinylfonnamide. The content of N-vinylformamide was divided by the original crude N-vinylformamide, and the resultant value was taken as the purity of N-vinylformamide.
Analysis system: LC analysis system manufactured by Shimadzu Corporation,
Column: ODP column (Shodex ODP 4.6 mm × 250 mm),
Eluent: 0.01 mol/L aqueous solution of disodium hydrogenphosphate,
Flow velocity: 1 mL/min,
Analysis temperature: 40°C,
Sample injection volume: 20 µL, and
Detector: UV detector (wavelength: 220 nm).

### (Measurement of Viscosity of Aqueous Solution of Vinylamine Unit-Containing Polymer)

The temperature of an aqueous solution of a vinylamine unit-containing polymer was set to 25°C. and the viscosity thereof was measured by using the Brookfield viscometer under the conditions of from 6 to 60 rpm and rotor No. 4. The viscosity was expressed as mPa·s (25°C).

### (Measurement of Molecular Weight of N-vinylformamide Unit-Containing Polymer and Vinylamine Unit-Containing Polymer)

A predetermined amount of an N-vinylformamide unit-containing polymer or an aqueous solution of a vinylamine unit-containing polymer was taken in a 25 mL volumetric flask and diluted with 50 vol% of a 0.5 mol/L aqueous solution of sodium chloride and 50 vol% of a 0.5 mol/L aqueous solution of acetic acid so that the concentration of the polymer became 0.1 mass%. The diluted solution was subjected to analysis by gel permeation chromatography (GPC) using the following apparatus under the following conditions to measure the weight average molecular weight.
Analysis system: GPC analysis system manufactured by Shimadzu Corporation,
Column: SEC column (OHpak SB-806M HQ 8 mm × 300 mm + OHpak SB-807 HQ 8 mm × 300 mm + OHpak SB-807 HQ 8 mm × 300 mm),
Eluent: 50 vol% of 0.5 mol/L aqueous solution of sodium chloride and 50 vol% of 0.5 mol/L aqueous solution of acetic acid
Flow velocity: 0.5 mL/min,
Analysis temperature: 40°C,
Sample injection volume: 400 µL, and
Detector: differential refractometer.

### (Method of Manufacturing N-vinylformamide)

By the method described in Example 1 of Patent Literature 1, N-vinylformamide was synthesized from formamide and acetaldehyde as starting materials via an intermediate N-methoxyethylformamide, thereby obtaining crude N-vinylformamide.

### (Method of Manufacturing N-vinylformamide Unit-Containing Polymer)

In 80 parts by mass of deionized water. 0.3 part by mass of polyethylene glycol (average molecular weight: 20,000) was dissolved, subsequently 30 parts by mass of crude N-vinylformamide was mixed with the solution, the pH of the mixture was adjusted with phosphoric acid, thereby obtaining a controlled monomer solution having a pH of 6.3. The pH was a value measured at 25°C.

This controlled monomer solution was cooled to 0°C, then transferred to an adiabatic reaction vessel equipped with a thermometer, and subjected to nitrogen aeration for 15 minutes, 1500 ppm (with respect to monomer) of 2,2'-azobis (2-amidinopropane) dihydrochloride (trade name: V-50 manufactured by Wako Pure Chemical Industries. Ltd.) and 300 ppm (with respect to monomer) of t-butyl hydroperoxide (trade name: PERBUTYL H-69 manufactured by NOF Corporation) were then added thereto as 10% aqueous solutions, and polymerization was then started by adding 300 ppm (with respect to monomer) of sodium hydrogensulfite (MBS) to the mixture as a 10 mass% aqueous solution.

After the temperature in the system reached the maximum temperature, the mixture was further held in the reaction vessel for 60 minutes, thereby obtaining an N-vinylformamide unit-containing polymer in the form of a massive aqueous gel. Thereafter, the N-vinylformamide unit-containing polymer was taken out from the reaction vessel, pulverized by using a meat chopper, dried in a forced-air drier at 110°C for 1.5 hours, and then pulverized to be formed into a powder shape.

### (Method of Manufacturing Aqueous Solution of Vinylamine Unit-Containing Polymer)

To an aqueous solution prepared by mixing 131.9 g of water, 5.6 g of a 48 mass% aqueous solution of sodium hydroxide, and 0.5 g of sodium dithionitc. 12 g of the powdery N-vinylformamide unit-containing polymer was added little by little and dissolved at 50°C over 2 hours, and the mixture was subjected to a hydrolysis treatment at 80°C over 3 hours, thereby obtaining an aqueous solution of a vinylamine unit-containing polymer.

### <Example 1>

### (Preservation of N-vinylformamide Unit-Containing Polymer)

Crude N-vinylformamide was obtained according to the "method of manufacturing N-vinylformamide" described above. The purity of N-vinylformamide in the crude N-vinylformamide was measured and found to be 92 mass%.

Subsequently, a powdery N-vinylformamide unit-containing polymer was obtained by using this crude N-vinylformamide according to the "method of manufacturing N-vinylformamide unit-containing polymer" described above. The time required until the temperature in the system reached the maximum temperature was 280 minutes. The weight average molecular weight of this polymer was measured and found to be 2,700,000.

This powdery N-vinylformamide unit-containing polymer was preserved at room temperature (maximum temperature: 30°C, average: 20°C to 28°C) for 30 days. The weight average molecular weight thereof was measured again and found not to be changed from that before preservation.

An aqueous solution of a vinylamine unit-containing polymer was obtained by using this powdery N-vinylformamide unit-containing polymer preserved according to the "method of manufacturing aqueous solution of vinylamine unit-containing polymer" described above. The viscosity of the aqueous solution was measured and found to be 15,000 mPa·s. In addition, the weight average molecular weight of the vinylamine unit-containing polymer was measured and found to be 2,600,000.

### <Comparative Example 1>

### (Preservation of N-vinylformamide)

Crude N-vinylformamide was obtained in the same manner as in Example 1. This crude N-vinylformamide was preserved at room temperature for 30 days as it was. The purity of N-vinylformamide in the crude N-vinylformamide after preservation was measured, and the purity decreased to 78 mass%.

Subsequently, a powdery N-vinylformamide unit-containing polymer was obtained by using this crude N-vinylformamide preserved according to the "method of manufacturing N-vinylformamide unit-containing polymer" described above. The time required until the temperature in the system reached the maximum temperature was 490 minutes to be greatly delayed as compared to that in Example 1. The weight average molecular weight of this polymer was measured and found to be 1,900,000, and this value was 30% lower than that of the N-vinylformamide unit-containing polymer in Example 1.

Subsequently, an aqueous solution of a vinylamine unit-containing polymer was obtained by using this powdery N-vinylformamide unit-containing polymer according to the "method of manufacturing aqueous solution of vinylamine unit-containing polymer" described above. The viscosity of the aqueous solution was measured and found to be 6,000 mPa·s, and this value was 60% lower than that in Example 1. In addition, the weight average molecular weight of the vinylamine unit-containing polymer was measured and found to be 1,800,000. and this value was 31% lower than that in Example 1.

### <Comparative Example 2>

### (Preservation of Aqueous Solution of Vinylamine Unit-Containing Polymer)

Crude N-vinylformamide was obtained and a powdery N-vinylformamide unit-containing polymer was obtained by using this crude N-vinylformamide in the same manner as in Example 1.

Subsequently, an aqueous solution of a vinylamine unit-containing polymer was obtained by using this powdery N-vinylformamide unit-containing polymer according to the "method of manufacturing aqueous solution of vinylamine unit-containing polymer" described above.

This aqueous solution of a vinylamine unit-containing polymer was preserved at room temperature for 30 days. The viscosity of the aqueous solution after preservation was measured and found to be 10.000 mPa·s, and this value was 33% lower than that in Example 1. In addition, the weight average molecular weight of the vinylamine unit-containing polymer was measured and found to be 2,100,000, and this value was 19% lower than that in Example 1.

### <Example 2>

### (Preservation of N-vinylformamide Unit-Containing Polymer)

The same operation as in Example 1 was conducted except that the preservation period of the N-vinylformamide unit-containing polymer was extended to 180 days.

The maximum temperature of room temperature at the time of preservation of the N-vinylformamide unit-containing polymer was 33°C (average: 20°C to 28°). The weight average molecular weight of the N-vinylformamide unit-containing polymer after preservation was measured again and found to be 2,600,000, this value was about 4% lower than that in Example 1, and a decrease in the molecular weight was thus suppressed low.

In addition, the viscosity of the aqueous solution of a vinylamine unit-containing polymer obtained was measured and found to be 14,500 mPa·s, this value was about 3% lower than that in Example 1, and a decrease in the viscosity was thus suppressed low. In addition, the weight average molecular weight of the vinylamine unit-containing polymer was measured and found to be 2,500,000, this value was about 4% lower than that in Example 1, and a decrease in the molecular weight was thus suppressed low.

### <Example 3>

### (Preservation of Aqueous Solution of N-vinylformamide Unit-Containing Polymer)

Crude N-vinylformamide was obtained and a powdery N-vinylformamide unit-containing polymer was obtained by using this crude N-vinylformamide in the same manner as in Example 1. With 131.9 g of water, 15 g of this polymer was mixed and dissolved at 50°C over 2 hours to obtain an aqueous solution of the N-vinylformamide unit-containing polymer.

This aqueous solution was preserved at room temperature for 180 days. The maximum temperature of room temperature was 33°C (average: 20°C to 28°). The weight average molecular weight of the N-vinylformamide unit-containing polymer after preservation was measured and found to be 2,400,000, this value was about 11% lower than that in Example 1, and a decrease in the molecular weight was thus suppressed relatively low although the molecular weight slightly decreased as compared to that of the powdery N-vinylformamide unit-containing polymer after preservation in

### Example 2.

Subsequently, an aqueous solution of a vinylamine unit-containing polymer was obtained by using this aqueous solution after preservation in the same manner as in the "method of manufacturing aqueous solution of vinylamine unit-containing polymer" described above except that the addition of water was omitted. The viscosity of the aqueous solution was measured and found to be 12,000 mPa·s, this value was about 20% lower than that in Example 1. and a decrease in the viscosity was thus suppressed relatively low. In addition, the weight average molecular weight of the vinylamine unit-containing polymer was measured and found to be 2.200,000, this value was about 15% lower than that in Example 1. and a decrease in the molecular weight was thus suppressed relatively low.

### <Comparative Example 3>

### (Preservation of N-vinylformamide)

The same operation as in Comparative Example 1 was conducted except that the preservation period of crude N-vinylformamide was extended to 180 days.

The maximum temperature of room temperature at the time of preservation of the crude N-vinylformamide was 33°C (average: 20°C to 28°C). The purity of N-vinylformamide in this crude N-vinylformamide was measured, and the purity was decreased to 42 mass%.

In addition, the time required until the temperature in the system reached the maximum temperature at the time of manufacture of the N-vinylformamide unit-containing polymer was 800 minutes to be further greatly delayed even as compared to that in Comparative Example 1. The weight average molecular weight of this polymer was measured and found to be 1,200,000, and this value was 56% lower than that of the N-vinylformamide unit-containing polymer in Example 1.

In addition, the viscosity of the aqueous solution of a vinylamine unit-containing polymer obtained was measured and found to be 2,000 mPa·s, and this value was 87% lower than that in Example 1. In addition, the weight average molecular weight of the vinylamine unit-containing polymer was measured and found to be 1,000,000, and this value was 62% lower than that in Example 1.

### <Comparative Example 4>

### (Preservation of Aqueous Solution of Vinylamine Unit-Containing Polymer)

The same operation as in Comparative Example 2 was conducted except that the preservation period of the aqueous solution of a vinylamine unit-containing polymer was extended to 180 days.

The viscosity of the aqueous solution of a vinylamine unit-containing polymer after preservation was measured and found to be 5,000 mPa·s, and this value was 67% lower than that in Example 1. The weight average molecular weight of the vinylamine unit-containing polymer was measured and found to be 1,550,000, and this value was 40% lower than that in Example 1.

### <Example 4>

### (Preservation of N-vinylformamide Unit-Containing Polymer)

The same operation as in Example 1 was conducted except that the preservation period of the N-vinylformamide unit-containing polymer was shortened to 15 days.

The maximum temperature of room temperature at the time of preservation of the N-vinylformamide unit-containing polymer was 31°C (average: 24°C to 29°C). The weight average molecular weight of the polymer after preservation was measured and found to be 2,700,000, and the molecular weight was not changed as compared to that in Example 1.

In addition, the viscosity of the aqueous solution of a vinylamine unit-containing polymer obtained was measured and found to be 14.700 mPa·s, this value was about 2% lower than that in Example 1, and a decrease in the viscosity was thus suppressed low. In addition, the weight average molecular weight of the vinylamine unit-containing polymer was measured and found to be 2,600,000, and the molecular weight was not changed as compared to that in Example 1.

### <Comparative Example 5>

### (Preservation of N-vinylformamide)

The same operation as in Comparative Example 1 was conducted except that the preservation period of the crude N-vinylformamide was shortened to 15 days.

The maximum temperature of room temperature at the time of preservation of the crude N-vinylformamide was 31°C (average: 24°C to 29°C). The purity of N-vinylformamide in this crude N-vinylformamide was measured, and the purity was decreased to 84 mass%.

In addition, the time required until the temperature in the system reached the maximum temperature at the time of manufacture of the N-vinylformamide unit-containing polymer was 410 minutes to be delayed. The weight average molecular weight of this polymer was measured and found to be 2,100,000, and this value was 22% lower than that of the N-vinylformamide unit-containing polymer in Example 1.

In addition, the viscosity of the aqueous solution of a vinylamine unit-containing polymer obtained was measured and found to be 10,000 mPa·s, and this value was 33% lower than that in Example 1. In addition, the weight average molecular weight of the vinylamine unit-containing polymer was measured and found to he 2,000,000, and this value was 23% lower than that in Example 1.

### <Comparative Example 6>

### (Preservation of Aqueous Solution of Vinylamine Unit-Containing Polymer)

The same operation as in Comparative Example 2 was conducted except that the preservation period of the aqueous solution of a vinylamine unit-containing polymer was shortened to 15 days.

The viscosity of the aqueous solution of a vinylamine unit-containing polymer after preservation was measured and found to be 12,000 mPa·s, and this value was 20% lower than that in Example 1. In addition, the weight average molecular weight of the vinylamine unit-containing polymer was measured and found to be 2,200,000, and this value was 15% lower than that in Example 1.

### <Example 5>

### (Preservation of N-vinylformamide Unit-Containing Polymer)

The same operation as in Example 1 was conducted except that the N-vinylformamide unit-containing polymer was preserved in an incubator at 40°C.

The weight average molecular weight of the polymer after preservation was measured and found to be 2,600,000, this value was about 4% lower than that m Example 1, and a decrease in the molecular weight was thus suppressed low.

In addition, the viscosity of the aqueous solution of a vinylamine unit-containing polymer obtained was measured and found to be 14,300 mPa·s, this value was 5% lower than that in Example 1, and a decrease in the viscosity was thus suppressed low. In addition, the weight average molecular weight of the vinylamine unit-containing polymer was measured and found to be 2,500,000, this value was about 4% lower than that in Example 1, and a decrease in the molecular weight was thus suppressed low.

### <Comparative Example 7>

### (Preservation of N-vinylformamide)

The same operation as in Comparative Example 1 was conducted except that N-vinylformamide was preserved in an incubator at 40°C.

The purity of N-vinylformamide in the crude N-vinylformamide after preservation was measured, and the purity was decreased to 39 mass%.

In addition, the time required until the temperature in the system reached the maximum temperature at the time of manufacture of the N-vinylformamide unit-containing polymer was 900 minutes to be greatly delayed as compared to that in Example 1. The weight average molecular weight of this polymer was measured and found to be 900,000, and this value was 67% lower than that of the N-vinylformamide unit-containing polymer in Example 1.

In addition, the viscosity of the aqueous solution of a vinylamine unit-containing polymer obtained was measured and found to he 1,500 mPa·s, and this value was 90% lower than that in Example 1. In addition, the weight average molecular weight of the vinylamine unit-containing polymer was measured and found to be 850,000, and this value was 67% lower than that in Example 1.

### <Comparative Example 8>

### (Preservation of Aqueous Solution of Vinylamine Unit-Containing Polymer)

The same operation as in Comparative Example 2 was conducted except that the aqueous solution of a vinylamine unit-containing polymer was preserved in an incubator at 40°C.

The viscosity of the aqueous solution of a vinylamine unit-containing polymer after preservation was measured and found to be 3,400 mPa·s, and this value was 77% lower than that in Example 1. In addition, the weight average molecular weight of the vinylamine unit-containing polymer was measured and found to he 1,200,000, and this value was 54% lower than that in Example 1.

### <Example 6>

### (Preservation of Monomer and Respective Polymers)

Crude N-vinylformamide was obtained and a powdery N-vinylformamide unit-containing polymer was obtained by using this crude N-vinylformamide in the same manner as in Example 1.

Subsequently, this powdery N-vinylformamide unit-containing polymer was preserved at room temperature for 15 days. The weight average molecular weight of the polymer after preservation was measured and found to be 2,600,000, this value was about 4% lower than that in Example 1, and a decrease in the molecular weight was thus suppressed low.

An aqueous solution of a vinylamine unit-containing polymer was obtained by using this powdery N-vinylformamide unit-containing polymer according to the "method of manufacturing aqueous solution of vinylamine unit-containing polymer" described above.

This aqueous solution of a vinylamine unit-containing polymer was preserved at room temperature for 5 days, and the viscosity of the aqueous solution after preservation was measured and found to be 14,500 mPa·s, this value was about 3% lower than that in Example 1, and a decrease in the viscosity was thus suppressed low. In addition, the weight average molecular weight of the vinylamine unit-containing polymer was measured and found to be 2,550,000. this value was about 2% lower than that in Example 1, and a decrease in the molecular weight was thus suppressed low.

### <Comparative Example 9>

### (Preservation of Monomer and Respective Polymers)

Crude N-vinylformamide was preserved at room temperature for 15 days in the same manner as in Comparative Example 5. The purity of N-vinylformamide in this crude N-vinylformamide was measured, and the purity was decreased to 82 mass%.

In addition, the time required until the temperature in the system reached the maximum temperature at the time of manufacture of the N-vinylformamide unit-containing polymer was 450 minutes to be delayed. The weight average molecular weight of this polymer was measured and found to be 2,000,000, and this value was 26% lower than that of the N-vinylformamide unit-containing polymer in Example 1.

The N-vinylformamidc unit-containing polymer obtained was preserved at room temperature for 5 days, and an aqueous solution of a vinylamine unit-containing polymer was then obtained according to the "method of manufacturing aqueous solution of vinylamine unit-containing polymer" described above. This aqueous solution of a vinylamine unit-containing polymer was preserved at room temperature for 5 days, and the viscosity of the aqueous solution after preservation was measured and found to be 8,000 mPa·s, and this value was 47% lower than that in Example 1. In addition, the weight average molecular weight of the vinylamine unit-containing polymer was measured and found to be 1,900,000, and this value was 27% lower than that in Example 1.

### <Comparative Example 10>

### (Preservation of Monomer and Respective Polymers)

The same operation as in Example 6 was conducted except that the preservation period of the N-vinylformamide unit-containing polymer was set to 5 days and the preservation period of the aqueous solution of a vinylamine unit-containing polymer was set to 15 days.

The viscosity of the aqueous solution of a vinylamine unit-containing polymer after preservation was measured and found to be 11,000 mPa·s, and this value was 27% lower than that in Example 1. In addition, the weight average molecular weight of the vinylamine unit-containing polymer was measured and found to be 2,150.000, and this value was 17% lower than that in Example 1.

### <Example 7>

### (Preservation of Powdery N-vinylformamide Unit-Containing Polymer under Vibration)

Crude N-vinylformamide was obtained in the same manner as in Example 1, and 10 g of this crude N-vinylformamide was added into a 50 ml Erlenmeyer flask. This was placed in a shaker and continuously shaken at room temperature for 30 days at a frequency of vibration of 20 Hz. The weight average molecular weight of the polymer after shaking was measured and found to be 2,600,000, this value was about 4% lower than that in Example 1, and a decrease in the molecular weight was thus suppressed low.

An aqueous solution of a vinylamine unit-containing polymer was obtained by using this powdery N-vinylformamide unit-containing polymer according to the "method of manufacturing aqueous solution of vinylamine unit-containing polymer" described above.

The viscosity of this aqueous solution of a vinylamine unit-containing polymer was measured and found to be 14.500 mPa·s, this value was about 3% lower than that in Example 1. and a decrease in the viscosity was thus suppressed low. In addition, the weight average molecular weight of the vinylamine unit-containing polymer was measured and found to be 2,550,000, this value was about 2% lower than that in Example 1, and a decrease in the molecular weight was thus suppressed low.

### <Comparative Example 11>

### (Preservation of N-vinylformamide under Vibration)

Crude N-vinylformamide was obtained in the same manner as in Example 1, and 10 g of this crude N-vinylformamide was added into a 50 ml Erlenmeyer flask. This was placed in a shaker and continuously shaken at room temperature for 30 days at a frequency of vibration of 20 Hz. The purity of N-vinylformamide in this crude N-vinylformamide was measured, and the purity was decreased to 70 mass%.

In addition, the time required until the temperature in the system reached the maximum temperature at the time of manufacture of the N-vinylformamide unit-containing polymer was 570 minutes to be delayed. The weight average molecular weight of this polymer was measured and found to be 1,750,000, and this value was 35% lower than that of the N-vinylformamide unit-containing polymer in Example 1.

In addition, the viscosity of the aqueous solution of a vinylamine unit-containing polymer obtained was measured and found to be 5,400 mPa·s, and this value was 64% lower than that in Example 1. In addition, the weight average molecular weight of the vinylamine unit-containing polymer was measured and found to be 1,600,000, and this value was 38% lower than that in Example 1.

### <Comparative Example 12>

### (Preservation of Aqueous Solution of Vinylamine Unit-Containing Polymer under Vibration)

Crude N-vinylformamide was obtained and a powdery N-vinylformamide unit-containing polymer was obtained by using this crude N-vinylformamide in the same manner as in Example 1.

Subsequently, an aqueous solution of a vinylamine unit-containing polymer was obtained by using this powdery N-vinylformamide unit-containing polymer according to the "method of manufacturing aqueous solution of vinylamine unit-containing polymer" described above.

Into a 50 ml Erlenmeyer flask, 10 g of this aqueous solution of a vinylamine unit-containing polymer was added. This was placed in a shaker and continuously shaken at room temperature for 30 days at a frequency of vibration of 20 Hz. The viscosity of the aqueous solution after shaking was measured and found to be 8.300 mPa·s, and this value was 45% lower than that in Example 1. In addition, the weight average molecular weight of the vinylamine unit-containing polymer was measured and found to be 1,950,000, and this value was 25% lower than that in Example 1.

### <Example 8>

### (Preservation of Powdery N-vinylformamide Unit-Containing Polymer under Vibration)

The same operation as in Example 7 was conducted except that the frequency of vibration was changed to 200 Hz. The weight average molecular weight of the polymer after shaking was measured and found to be 2,600,000, this value was about 4% lower than that in Example 1, and a decrease in the molecular weight was thus suppressed low.

In addition, the viscosity of the aqueous solution of a vinylamine unit-containing polymer obtained was measured and found to be 14,200 mPa·s, this value was about 5% lower than that in Example 1, and a decrease in the viscosity was thus suppressed low. In addition, the weight average molecular weight of the vinylamine unit-containing polymer was measured and found to be 2,450,000, this value was about 6% lower than that in Example 1, and a decrease in the molecular weight was thus suppressed low.

### <Comparative Example 13>

### (Preservation of N-vinylformamide under Vibration)

The same operation as in Comparative Example 11 was conducted except that the frequency of vibration was changed to 200 Hz. The purity of N-vinylformamide in the crude N-vinyltormamide after shaking was measured, and the purity was decreased to b2 mass%.

In addition, the time required until the temperature in the system reached the maximum temperature at the time of manufacture of the N-vinylformamide unit-containing polymer was 620 minutes to be delayed. The weight average molecular weight of this polymer was measured and found to be 1,500,000, and this value was 44% lower than that of the N-vinylformamide unit-containing polymer in Example 1.

In addition, the viscosity of the aqueous solution of a vinylamine unit-containing polymer obtained was measured and found to be 4,400 mPa·s, and this value was 60% lower than that in Example 1. In addition, the weight average molecular weight of the vinylamine unit-containing polymer was measured and found to be 1,450,000, and this value was 44% lower than that in Example 1.

### <Comparative Example 14>

### (Preservation of Aqueous Solution of Vinylamine Unit-Containing Polymer under Vibration)

The same operation as in Comparative Example 12 was conducted except that the frequency of vibration was changed to 200 Hz. The viscosity of the aqueous solution after shaking was measured and found to be 6,800 mPa·s, and this value was 55% lower than that in Example 1. In addition, the weight average molecular weight of the vinylamine unit-containing polymer was measured and found to be 1,850,000, and this value was 29% lower than that in Example 1.

The respective preservation conditions and results are presented in Tables 1 and 2.

**[Table 1]**

| | Vibration | Immediately after manufacture of N-vinylformamide → immediately before manufacture of N-vinylformamide unit-containing polymer: preservation period (1) | | Immediately after manufacture of N-vinylformamide unit-containing polymer → immediately before manufacture of aqueous solution of vinylamine unit-containing polymer: preservation period (2) | | | Immediately after manufacture of aqueous solution of vinylamine unit-containing polymer → immediately before use thereof: preservation period (3) | |
|---|---|---|---|---|---|---|---|---|
| | | Preservation temperature | Preserv ation period | Preservation form | Preservation temperature | Preservation period | Preservation temperature | Preservation period |
| Example 1 | 0 Hz | - | - | Powder | Room temperature | 30 days | - | - |
| Example 2 | 0 Hz | - | - | Powder | Room temperature | 180 days | - | - |
| Example 3 | 0 Hz | - | - | Aqueous solution | Room temperature | 180 days | - | - |
| Example 4 | 0 Hz | - | - | Powder | Room temperature | 15 days | - | - |
| Example 5 | 0 Hz | - | - | Powder | 40°C | 30 days | - | - |
| Example 6 | 0 Hz | - | - | Powder | Room temperature | 15 days | Room temperature | 5 days |
| Example 7 | 20 Hz | - | - | Powder | Room temperature | 30 days | - | - |
| Example 8 | 200 Hz | - | - | Powder | Room temperature | 30 days | - | - |
| Comparative Example 1 | 0 Hz | Room temperature | 30 days | - | - | - | - | - |
| Comparative Example 2 | 0 Hz | - | - | - | - | - | Room temperature | 30 days |
| Comparative Example 3 | 0 Hz | Room temperature | 180 days | - | - | - | - | - |
| Comparative Example 4 | 0 Hz | - | - | - | - | - | Room temperature | 180 days |
| Comparative Example 5 | 0 Hz | Room temperature | 15 days | - | - | - | - | - |
| Comparative Example 6 | 0 Hz | - | - | - | - | - | Room temperature | 15 days |
| Comparative Example 7 | 0 Hz | 40°C | 30 days | - | - | - | - | - |
| Comparative Example 8 | 0 Hz | - | - | - | - | - | 40°C | 30 days |
| Comparative Example 9 | 0 Hz | Room temperature | 15 days | Powder | Room temperature | 5 days | Room temperature | 5 days |
| Comparative Example 10 | 0 Hz | - | - | Powder | Room temperature | 5 days | Room temperature | 15 days |
| Comparative Example 11 | 20 Hz | Room temperature | 30 days | - | - | - | - | - |
| Comparative Example 12 | 20 Hz | Room temperature | 30 days | - | - | - | - | - |
| Comparative e Example 13 | 200 Hz | - | - | - | - | - | Room temperature | 30 days |
| Comparative Example 14 | 200 Hz | - | - | - | - | - | Room temperature | 30 days |

**[Table 2]**

| | Purity of N-vinylformamide | Time required until temperature in system reaches maximum temperature | Molecular weight of N-vinylformamide unit-containing polymer | Viscosity of aqueous solution of vinylamine unit-containing polymer (rate of decrease [with respect to Example 1]) | Molecular weight of vinylamine unit-containing polymer (rate of decrease with respect to Example 1]) | Total preserv ation period |
|---|---|---|---|---|---|---|
| Example 1 | 92 mass% | 280 minutes | 2,700,000 | 15,000 mPa·s | 2,600,000 (-) | 30 days |
| Example 2 | 92 mass% | 280 minutes | 2,600,000 | 14,500 mPa·s (3%) | 2,500,000 (4%) | 180 days |
| Example 3 | 92 mass% | 280 minutes | 2,400,000 | 12,000 mPa·s (20%) | 2,200,000 (15%) | 180 days |
| Example 4 | 92 mass% | 280 minutes | 2,700,000 | 14,700 mPa·s (2%) | 2,600,000 (6%) | 15 days |
| Example 5 | 92 mass% | 280 minutes | 2,600,000 | 14.300 mPa·s (5%) | 2,500,000 (4%) | 30 days |
| Example 6 | 92 mass% | 280 minutes | 2,600,000 | 14,500 mPa·s (3%) | 2,550,000 (2%) | 20 days |
| Example 7 | 92 mass% | 280 minutes | 2,600,000 | 14,500 mPa· s (3%) | 2,550,000 (2%) | 30 days |
| Example 8 | 92 mass% | 280 minutes | 2,600,000 | 14,200 mPa· s (5%) | 2,450,000 (6%) | 30 days |
| Comparative Example 1 | 78 mass% | 490 minutes | 1,900,000 | 6,000 mPa·s | 1,800,000 (31%) | 30 days |
| Comparative Example 2 | 92 mass% | 280 minutes | 2,700,000 | 10,000 mPa·s (33%) | 2,100,000 (19%) | 30 days |
| Comparative Example 3 | 42 mass% | 800 minutes | 1,200,000 | 2,000 mPa·s (87%) | 1,000,000 (62%) | 180 days |
| Comparative Example 4 | 92 mass% | 280 minutes | 2,700,000 | 5,000 mPa·s ( 67%) | 1,550,000 (40%) | 180 days |
| Comparative Example 5 | 84 mass% | 410 minutes | 2,100,000 | 10,000 mPa·s (33%) | 2,000,000 (23%) | 15 days |
| Comparative Example 6 | 92 mass% | 280 minutes | 2,700,000 | 12,000 mPa·s (20%) | 2,200,000 (15%) | 15 days |
| Comparative Example 7 | 39 mass% | 900 minutes | 900,000 | 1,500 mPa· s (90%) | 850,000 (67%) | 30 days |
| Comparative Example 8 | 92 mass% | 280 minutes | 2,700,000 | 3,400 mPa·s (77%) | 1,200,000 (54%) | 30 days |
| Comperative Example 9 | 82 mass% | 450 minutes | 2,000,000 | 8,000 mPa·s (47%) | 1,900,000 (27%) | 25 days |
| Comparative Example 10 | 92 mass% | 280 minutes | 2,700,000 | 11,000 mPa·s (27%) | 2,150,000 (17%) | 20 days |
| Comparative Example 11 | 70 mass% | 570 minutes | 1,750,000 | 5,400 mPa·s (64%) | 1,600,000 (38%) | 30 days |
| Comparative Example 12 | 92 mass% | 280 minutes | 2,700,000 | 8,300 mPa·s (45%) | 1,950,000 (25%) | 30 days |
| Comparative Example 13 | 62 mass% | 620 minutes | 1,500,000 | 4,400 mPa·s (60%) | 1,450,000 (44%) | 30 days |
| Comparative Example 14 | 92 mass% | 280 minutes | 2,700,000 | 6,800 mPa s (55%) | 1,850,000 (29%) | 30 days |

In Examples 1 to 4 in which the preservation period of the N-vinylformamide unit-containing polymer was the longest among N-vinylformamide, the N-vinylformamide unit-containing polymer, and the aqueous solution of a vinylamine unit-containing polymer, there was not a great decrease in the molecular weight of the N-vinylformamide unit-containing polymer regardless of the preservation conditions. A great decrease in the molecular weight was not observed in the vinylamine unit-containing polymer obtained by hydrolyzing the N-vinylformamide unit-containing polymer after preservation as well.

On the other hand, in Comparative Examples 1, 3, 5, and 7 in which the preservation period of N-vinylformamide was set to be the longest, N-vinylformamide was decomposed during preservation and the purity of N-vinylformamide in crude N-vinylformamide greatly decreased. Hence, the polymerization time was greatly delayed when polymerizing N-vinylformamide and the molecular weights of the N-vinylformamide unit-containing polymer after polymerization and the vinylamine unit-containing polymer after hydrolysis also greatly decreased.

In addition, in Comparative Examples 2, 4, 6. and 8 in which the preservation period of the aqueous solution of a vinylamine unit-containing polymer was set to be the longest, the viscosity of the aqueous solution and the molecular weight of the vinylamine unit-containing polymer greatly decreased.

In addition, as indicated in Example 6, a great decrease in the molecular weight of the polymer was not observed when the preservation period of the N-vinylformamide unit-containing polymer was longer than the preservation periods of N-vinylformamide and the aqueous solution of a vinylamine unit-containing polymer in the case of providing a preservation period for the monomer and the respective polymers.

On the other hand, as indicated in Comparative Example 9 and Comparative Example 10, a delay in the polymerization time and a decrease in the molecular weight of the polymer were confirmed when the preservation periods of N-vinylformamide and the aqueous solution of a vinylamine unit-containing polymer were longer than the preservation period of the N-vinylformamide unit-containing polymer.

In addition, as indicated in Example 7 and Example 8, a great decrease in the molecular weight of the polymer was not observed when the preservation period of the N-vinylformamide unit-containing polymer was longer than the preservation periods of N-vinylformamide and the aqueous solution of a vinylamine unit-containing polymer even under the condition of applying vibrations.

On the other hand, as indicated in Comparative Examples 11 to 14, a delay in the polymerization time and a decrease in the molecular weight of the polymer were confirmed and a more remarkable decrease in stability as compared to usual preservation in which vibrations were not applied was observed when the preservation periods of N-vinylformamide and the aqueous solution of a vinylamine unit-containing polymer were longer than the preservation period of the N-vinylformamide unit-containing polymer under the condition of applying vibrations.

From the facts described above, it has been confirmed that an aqueous solution of a vinylamine unit-containing polymer can be most stably used when the period to preserve an N-vinylformamide unit-containing polymer is set to be the longest in a case in which N-vinylformamide is manufactured and an aqueous solution of a vinylamine unit-containing polymer is manufactured via an N-vinylformamide unit-containing polymer.

### INDUSTRIAL APPLICABILITY

According to the invention, the method of using an aqueous solution of a vinylamine unit-containing polymer can be utilized in various fields such as aggregating agents and fiber treatment agents in the water treatment field and the wastewater treatment including papermaking chemicals in the papennaking industry.

According to the use method of the invention, it is possible to suppress the influence of preservation conducted during the period from the manufacture of N-vinylformamide to the manufacture of an aqueous solution of a vinylamine unit-containing polymer via an N-vinylformamide unit-containing polymer and the use thereof on the quality of the aqueous solution of a vinylamine unit-containing polymer and to stably use the aqueous solution of a vinylamine unit-containing polymer.

## Claims

1. A method of using an aqueous solution of a vinylamine unit-containing polymer, the method comprising:
manufacturing N-vinylformamide;
manufacturing an N-vinylformamide unit-containing polymer by polymerizing a monomer component containing the N-vinylformamide;
manufacturing an aqueous solution of a vinylamine unit-containing polymer by hydrolyzing the N-vinylformamide unit-containing polymer by using an acid or a base and water; and
setting a preservation period (2) of the N-vinylformamide unit-containing polymer from immediately after manufacture of the N-vinylformamide unit-containing polymer to immediately before start of manufacture of the aqueous solution of a vinylamine unit-containing polymer to be longer than a preservation period (3) of the aqueous solution of a vinylamine unit-containing polymer from immediately after manufacture of the aqueous solution of a vinylamine unit-containing polymer to immediately before use of the aqueous solution of a vinylamine unit-containing polymer when using the aqueous solution of a vinylamine unit-containing polymer.

2. A method of using an aqueous solution of a vinylamine unit-containing polymer, the method comprising:
manufacturing N-vinylformamide;
manufacturing an N-vinylformamide unit-containing polymer by polymerizing a monomer component containing the N-vinylformamide;
manufacturing an aqueous solution of a vinylamine unit-containing polymer by hydrolyzing the N-vinylformamide unit-containing polymer by using an acid or a base and water; and
setting a preservation period (2) of the N-vinylformamide unit-containing polymer from immediately after manufacture of the N-vinylformamide unit-containing polymer to immediately before start of manufacture of the aqueous solution of a vinylamine unit-containing polymer to be longer than a preservation period (1) of the N-vinylformamide from immediately after manufacture of the N-vinylformamide to immediately before start of manufacture of the N-vinylformamide unit-containing polymer and a preservation period (3) of the aqueous solution of a vinylamine unit-containing polymer from immediately after manufacture of the aqueous solution of a vinylamine unit-containing polymer to immediately before use of the aqueous solution of a vinylamine unit-containing polymer when using the aqueous solution of a vinylamine unit-containing polymer.

3. The use method according to claim 1, wherein a sum of the preservation periods (2) and (3) is 10 days or longer.

4. The use method according to claim 2, wherein a sum of the preservation periods (1), (2), and (3) is 10 days or longer.

5. The use method according to any one of claims 1 to 4, wherein a powdery substance is manufactured as the N-vinylformamide unit-containing polymer by polymerizing the monomer component and then drying a resultant substance and preserved during the preservation period (2).

6. The use method according to any one of claims 1 to 5, wherein a preservation atmosphere of the N-vinylformamide unit-containing polymer is a temperature of 25°C or higher in at least a part of the preservation period (2).

7. The use method according to any one of claims 1 to 6, wherein a weight average molecular weight of the N-vinylformamide unit-containing polymer is 100,000 or more.

8. The use method according to any one of claims 1 to 7, wherein the hydrolysis is conducted in an aqueous solution of the N-vinylformamide unit-containing polymer in the presence of a strong base.

9. The use method according to any one of claims 1 to 8, wherein a powdery substance is manufactured as the N-vinylformamide unit-containing polymer and transported to another place while applying a vibration of from 0.1 to 400 Hz to the substance during the preservation period (2), the N-vinylformamide unit-containing polymer is prepared into an aqueous solution, the hydrolysis is conducted in the aqueous solution, and a sum of the preservation periods (1), (2), and (3) is 10 days or longer.
